# EUROPEAN PATENT APPLICATION

(11) **EP 1 354 589 A1**
(43) Date of publication of application: **22.10.2003**
(21) Application number: 01272244.3
(22) Date of filing: 26.11.2001
(51) Int. Cl.: A61K 31/7004, A61K 31/7024, A61K 31/7028, A61P 9/00, A61P 35/04

(54) **ANGIOGENESIS INHIBITORS**

(30) Priority: 22.12.2000 JP 2000390095
(71) Applicant: JAPAN SCIENCE AND TECHNOLOGY CORPORATION, Kawaguchi-shi, Saitama 332-0012 (JP)
(72) Inventor: AKIYAMA, Shin-ichi, Kagoshima-shi, Kagoshima 891-0103 (JP); FURUKAWA, Tatsuhiko, Kagoshima-shi, Kagoshima 891-0175 (JP); UCHIMIYA, Hiroshi, Kagoshima-shi, Kagoshima 891-0175 (JP)
(74) Representative: Woods, Geoffrey Corlett
(86) International application number: JP0110265
(87) International publication number: WO02051423

(57) **Abstract**

An angiogenesis inhibitor and drug inhibiting the tumor metastasis exasperated by TP which comprises, as an effective component, at least one kind of compound selected from the group consisting of 2-deoxy-L-ribose, 2-deoxy-L-ribose derivatives whose hydroxyl group is methylated and/or acylated and 2-deoxy-D-ribose derivatives whose hydroxyl group is methylated and/or acylated.

## Description

### FIELD OF THE INVENTION

The present invention relates to an inhibitor for growth, invasion or metastasis of tumor using at least one compound selected from the group consisting of 2-deoxy-L-ribose, methylated and/or acylated derivatives of 2-deoxy-L-ribose and 2-deoxy-D-ribose derivatives whose hydroxyl group is methylated and/or acylated which has an activity to inhibit the angiogenesis which provides oxygen and nourishment to a tumor such as cancer.

The present invention is different from the anticancer agent which directly inhibit the growth of cancer cells or kill the cancer cells. For the metastasis of cancer, there are following processes, that is, detachment of the cancer cells from the tumor solid mass, invasion into blood vessel, transportation in blood vessel, adhesion to basement membrene of blood vessel, escaping to the outside of blood vessel and growth in metastasized organ. Therefore, an inhibiting agent for metastasis of cancer is an agent which inhibits anyone of processes mentioned above, [Bioscience Series, "Mallignantation and metastasis of cancer", edited by Motoharu Shimizu, Chugai Medical, 1993] , and is an agent to inhibit the metastasis of cancer by using an activated subject which inhibits angiogenesis.

### BACK GROUND OF THE INVENTION

The process in which a new blood vessel is produced is called as angiogenesis. It is already known that the angiogenesis is deeply concerned with various illnesses. That is, the angiogenesis is concerned with illness such as cancer, diabedic retinopathy, age-relating macula lutea degenaration, psoriasis or rheumatoid arthritis. In particular, recently, the investigation regarding to angiogenesis in cancer is largely paid attention. When cancer cell is grown to 1-2 mm³ size, it needs much more oxygen and nourishment to grow further larger. The cancer cells discharges a factor called as an angiogenesis factor which causes of angiogenesis. Said factor demands the formation of a new blood vessel from a near blood vessel and leads in a new blood vessel to a tumor, and by using oxygen and nourishment contained in blood, the growing rate can be suddenly increased. Further, the metastasis of cancer becomes possible through the microvessel in tumor.

Concerning such an important role of the angiogenesis in cancer, the development of an agent to inhibit growth, inversion or metastasis of cancer by inhibiting the angiogenesis of cancer so as to starve out the cancer is a pressing need. In fact, many agents which inhibit angiogenesis are already developed and actively applied to the clinical medicine therapy, however, there is no developed agent which is admitted as an inhibitor for angiogenesis up to the present. The reason why can be illustrated as follows, that is, the production or use of it is not so easy because it is a peptide, or there is a generation of a harmful side effect. The development of an inhibitor of lower molecular weight for angiogenesis which has less harmful side effect and can be produced and used easily is expected.

As mentioned above, the development of a therapy agent aiming prevention of angiogenesis has a possibility to inhibit not only the growth but also the metastasis of an origin tumor. The inhibitor for angiogenesis differs from the conventional anticancer agent from the view point that it does not aim the cellcide effect, but aims to shutter the provision of oxygen and nourishment to a tumor and has a possibility to coexist with a cancer by inhibiting a growth and an metastasis of a tumor. This concept is called as a dormancy therapy for cancer, and angiostatin is found out by Dr. Folkman of U.S.A. and said concept is advocated. When the inhibitor for angiogenesis is compared with the anticancer agent, it has an advantage that the harmful side effect is weak because it does not directly kill a cell, the resistance to the inhibitor is not generated so easily and the inhibitor has the wider anticancer spectrum. Therefore, it has an advantage that the safe and long term prescribing is possible because the load to a client who has a cancer is lighter.

As the inhibitors for angiogenesis which are being tested on clinical trial, an agent which inhibits the intracellular signal transduction of endothelial cells, an inhibitor for basement membrene degradation enzyme and an agent whose inhibiting mechanism is unknown can be mentioned. Among these inhibitors, an antibody or a peptide has a problem in convenience for actual use and an inhibitor for basement membrene degradation enzyme has a problem of causing myalgia which is an unexpected harmful side effect. An agent which peculiarly inhibits KDR/F1k-1 thyrosine kinase, which is a receptor of vascular endothelial growth factor (VEGF), is expected to inhibit only angiogenesis by VEGF. The overexpression of an enzyme tymidine phosphorylase (TP) involved in metabolism of tymidine, a kind of pyrimidine nucleoside, in various kinds of cancer, is observed. In these kinds of cancer, activity or expression of TP is correlated with the numbers of microvessels, and TP acts an important role for the angiogenesis of these cancers. In the cases of colorectal, differentiated gastric or renal cancer, TP was a prognosis factor.

It can be concerned that the growth or metastasis of these cancers can be suppressed by inhibiting the angiogenesis by TP. The inventors of the present invention have recently developed an inhibitor for enzyme activity, termed TPI. TPI can suppress the growth and metastasis of TP-expressing tumors. TPI has a possibility to increase the plasma level of tymidine that may cause undesirable side effect. The down stream mediator carrying the angiogenesis by TP is 2-deoxy-D-ribose, a degradation product of thymidine, which is a substrate of TP (refer to Nature 368, 198, 1994). A lower molecular weight compound which specifically inhibits the function of 2-deoxy-D-ribose is expected to inhibit the angiogenesis by TP more specifically.

The subject of the present invention is to provide an activated lower molecule weight compound which inhibits angiogenesis.

In varions kinds of cancers, the expression of an enzyme TP relating to metabolism of pyrimidine nucleoside is enhanced. TP is the same protein to platelet-derived endothelial cell growth factor PD-ECGF, which is an angiogenesis factor.

Since the enzyme activity is needed for the angiogenesis by TP, the inventors of the present invention investigated whether there is a substance possessing the angiogenesis activity in degradation products of tymidine, which is the substrate of TP, or not. And found out that 2-deoxy-D-ribose possesses the angiogenesis activity, further, 2-deoxy-L-ribose, which is a stereoisomer of said compound, inhibits angiogenesis by TP. Thus the subject of the present invention is solved.

### DISCLOSURE OF THE INVENTION

The first one of the present invention is an inhibitor for angiogenesis comprising, as an effective component, at least one kind of compound selected from the group consisting of 2-deoxy-L-ribose, 2-deoxy-L-ribose derivatives whose hydroxyl group is methylated and/or acylated and 2-deoxy-D-ribose derivatives whose hydroxyl group is methylated and/or acylated.

The second one of the present invention is a formulation comprising, as an effective component for inhibiting function of angiogenesis, at least one kind of compound selected from the group consisting of 2-deoxy-L-ribose, 2-deoxy-L-ribose derivatives whose hydroxyl group is methylated and/or acylated and 2-deoxy-D-ribose derivatives whose hydroxyl group is methylated and/or acylated.

The third one of the present invention is a formulation for supressing growth, invasion metastasis of tumor comprising, as an effective component for inhibiting function of angiogenesis, at least one kind of compound selected from the group consisting of 2-deoxy-L-ribose, 2-deoxy-L-ribose derivatives whose hydroxyl group is methylated and/or acylated and 2-deoxy-D-ribose derivatives whose hydroxyl group is methylated and/or acylated.

### BRIEF ILLUSTRATION OF THE DRAWINGS

Fig. 1 shows the inhibiting inhibition of the bovine aortic endothelial cells by 2-deoxy-L-ribose.
Fig. 2 shows the microscopic observation of inhibiting effect of angiogenesis by 2-deoxy-L-ribose.
Fig.3 shows the suppression of TP-dependent liver matastasis of tumor cells to liver by by 2-deoxy-L-ribose.

### THE BEST EMBODYMENT TO CARRY OUT THE INVENTION

The present invention will be illustrated more in detail.
A. As the lower molecule weight compound which specifically inhibits the angiogenesis action of 2-deoxy-D-ribose following compounds can be mentioned,
   (1) 2-deoxy-L-ribose, 2-deoxy-L-ribose whose hydroxyl group is partially or fully methylated and/or acylated, for example, acetylated or benzoylated 2-deoxy-L-ribose and
   (2) 2-deoxy-D-ribose whose hydroxyl group is partially or fully methylated and/or acylated, for example, acetylated or benzoylated 2-deoxy-L-ribose.
      In particular, the compounds of said (1) group can be mentioned as the desirable compounds.
B. Since the angiogenesis inhibitor and the medicine formulation of the present invention are characterized as less toxicity and less harmful side effect, they can be applied to prevention and/or therapy of various diseases of mammals (for example, human, cow, horse, dog, cat, monkey, mouse or rat, in particular human), further, long term prescribing is possible.

The angiogenesis inhibitor and the medicine formulation of the present invention can be used by crude powder, however, in general, is formed to a formulation using following supports for medicine formulation voluntarily by voluntary amount according to the conventional method.
As the mentioned "supports for medicine formulation", for example, an excipient, a thickner, a decaying agent, a dispersant, a dispersion promoter, a solvent (for example, injection water, alcohol, propyleneglycol, macrogoal, sesame oil, corn oil or others), a dispersant (for example, Tween80, HCO60, polyethyleneglycol, carboxymethylcellulose or sodium alginate), a dissolving adjuvant (for example, polyethyleneglicol, propyleneglycol, D-mannitol, benzylbenzoate, ethanol, trisaminomethane, sodium carbonate or sodium citrate), a suspending agent (for example, triethanolaminestearate, sodium lauryl sulfates benzarconium chloride, polyvinyl alcohol, polyvinyl pyrrolidone or hydroxymethylcellulose), a painless agent (for example, benzyl alcohol), an isotonic agent (for example sodium chloride or glycerin), a buffering agent (for example, phosphate, acetate, carbonate or citrate), a slipping agent (for example, magnesium stearate, calcium stearate, talc, starches or sodium benzoate), a coloring agent (for example, tar pigment, caramel, iron sesquioxide, titanium oxide or riboflavins), a corrigent (for example, sweetners or fragrants), a stabilizer (for example, sodium sulfite or ascorbic acid), a preserving agent (for example, parabens or sorbic acid), in vivo decomposing high polymer compound (for example, lactic acid-glycolic acid copolymer, lactic acid-butylic acid copolymer), cyclodextrin (for example, maltosyl- β -cyclodextrin, maltosyl- β -cyclodextrin caboxylic acid), sodium hydroxide and migliols can be mentioned.

The preparation method of the formulation is illustrated as follows, however, not intending to be limited to this method.

For the preparation of a tablet, following process is used. The angiogenesis inhibiting substance or salt of said substance as is or the homogeneous mixture prepared by adding a excipient, a bonding agent, a decaying agent or other appropriate additives to said substance is granulated by an adequate method, further, a slipping agent or others are added and molded by compression. Then, when need is arisen, it is possible to coat the surface of tablet by adequate coating agent aiming the tablet to be solved in an intestine or aiming the tablet to be long lasting.

For the preparation of injection liquid, following process is used. To the definite amount of angiogenesis inhibiting substance or salt of said substance a stabilizer, a dissolving adjuvant, a dispersant, an emulsifier, a buffering agent, a preserving agent, cyclodextrin, sodium hydroxide or others are added in case of necessity, dissolved or suspended or emulsified in injection water and adjust to the definite amount.

The angiogenesis inhibitor and medicine formulation of the present invention are desirable to be dosed for oral administration (including slow releasing) or by non oral dosage, namely, intravenous administration (including bolus, infusion and inclusion compound), subcutaneous and intramascular injection (including bolus, infusion and slow releasing), intrapercutaneous or intratumor administration or proximal administration can be mentioned. The dosis of those angiogenesis inhibitors and medicine formulations are changeable according to the selected compound, selected animal species as the object for administration and the number of the administration times, however, display broadly its effectiveness.

In the case of oral administration of said formulations, the ordinary doses for one day is from approximately 0.01mg/kg weight to approximately 100mg/kg weight, desirably from approximately 0.1mg/kg weight to 40mg/kg weight more desirably from approximately 1mg/kg weight to 20mg/kg weight by effective dose of angiogenesis inhibiting substance.

The actual doses of the effective component is to be decided according to the selection of the effective component, the form of each formulation, age of patients, weights of patients, sex of patients, the degree of a disease, method for dosage, term and interval of dosage, and can be changed on occasion by the judgement of a medical doctor.

### EXAMPLES

The present invention will be illustrated more in detail by following Examples, however, these Examples are intending to make more clear the usefulness of the present invention and not intending to limit the scope of the claims of the present invention.

### Example 1

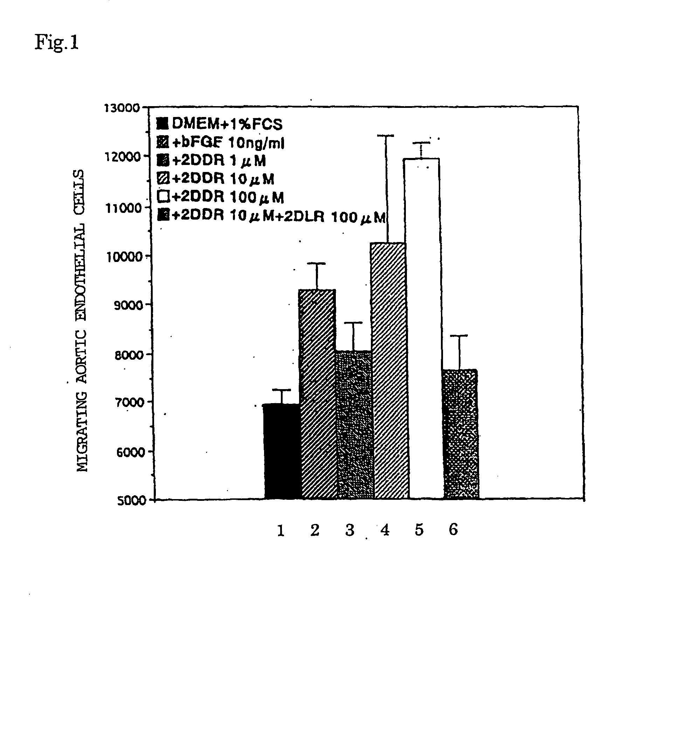

The experimental results of the effect of 2-deoxy-L-ribose on the chemotaxis of BAE cell (endothelial cells of bovine aorta) are shown in Fig.1.
The numbers of inoculated cell 400000, incubation 4.5hours
Chamber coated by type 1 collagen: MTT method

From left side, 1: DMEM+1%FCS (fetal calf serum)
2: 1+bFGF (angiogenesis factor) 10ng/mL,
3: 1+2DDR (2-deoxy-D-ribose) 1µM
4: 1+2DDR 10µM
5: 1+2DDR 100µM
6: 1+2DDR 10µM+2DLR100µM

The effect of 2DLR (2-deoxy-D-ribose) is obvious from the comparison between the date 4 and 6.

### Example 2

### Preparation of the formulation

150mg of 2-deoxy-L-ribose is dissolved into 30mL of physiological saline and
the injection liquid is prepared.

### Experimental Example

KB cells overpressing TP (KB/TP) prepared by transfection of TPcDNA into KB cells and TP-negative KB cells (KB/CV) prepared by transfection of an empty vector are rinsed by phosphate buffered saline PBS for two times, each 1 × 10⁵ numbers are contained into a Millipore chamber (10mm diameter, 2mm thickness, pore size of filter is 0.22µm) and inserted into dorsal subcutaneous of a male BALB/c mouse of 6-7 weeks age.

2-deoxy-L-ribose (100mg/kg/day) is administered into abdominal cavity every 12hours for 4 days. After 4 days from the implanting, the Millipore chamber is removed, and numbers of angiogenesis (longer than 3mm and showing zig-zag pattern) were counted using a microscope.

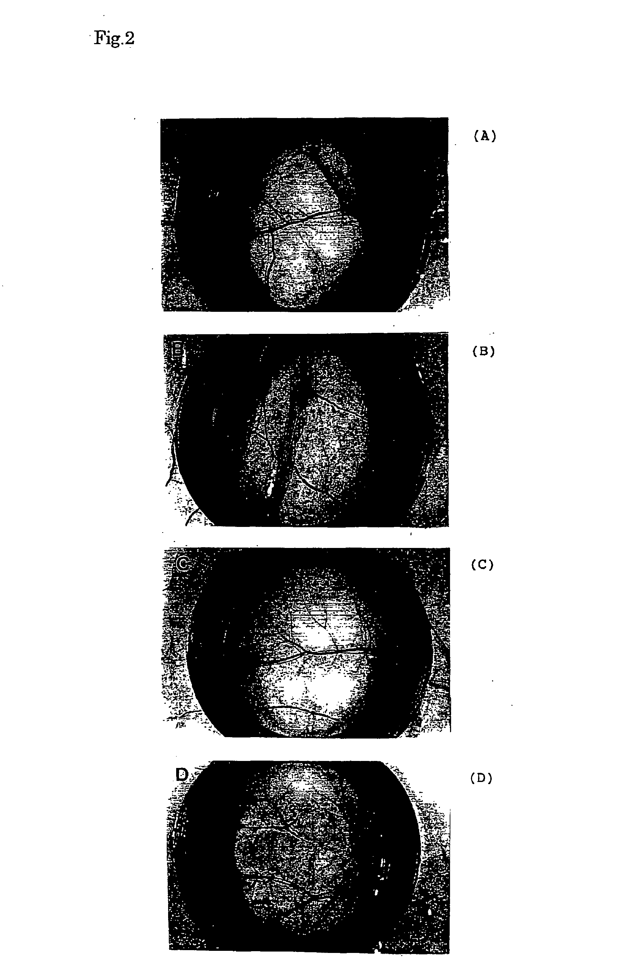

Results by microscope observation are shown in Fig.2 (picture A, B, C and D).

The number of angiogenesis by KB/TP cell (B) is 4.33 and that of KB/CV cell (B) is 2.67. When 2-deoxy-L-ribose is administereded, that of KB/TP cell (D) becomes to 2.83 and that of KB/TP cell (E) becomes to 2.83. These results obviously show that 2-deoxy-L-ribose is perfectly supressing the angiogenesis by TP.

### Example 3

A Balb/nude mouse of 7-8 weeks age is anesthetized by Nembutal (pentobarbital) and abdomen is slightly incised so as to observe the spleen. 1 × 10⁵ numbers of KB/CV cells or KB/TP cells used in Example 2 is injected into the spleen. After one minute, the spleen is excised and the incised position is sutured. 2-deoXy-L-ribose (100mg/kg/day) is injected to subcutaneous by two times (morning and evening) every day or one time every other day. To the control group, same amount of physiological saline is injected. After 4 weeks from therapy starting, abdomen is incised, the liver is excised and the number of metastatic nodules are counted.

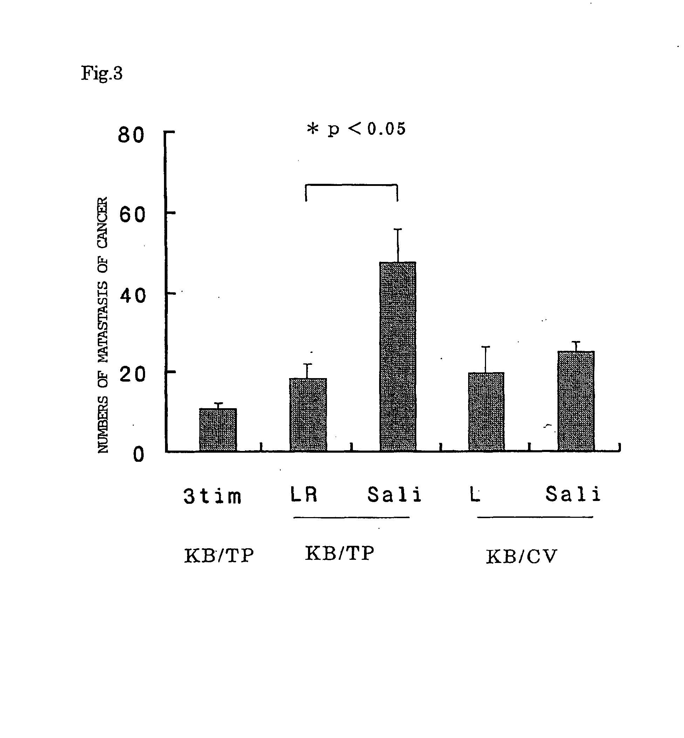

Fig.3 shows the averaged number of metastatic nodules when the experiments are carried out by 6 mice in one group. The number of metastatic lesions of KB/TP cell is obviously larger than those of KB/CV cell. And the number of metastatic noduless of the group which after KB/TP cell is injected into the spleen, 2-deoxy-L-ribose (100mg/kg/day) is injected to subcutaneous by two times (morning and evening) every day (LR) or the group which 2-deoxy-L-ribose (100mg/kg/day) is injected to subcutaneous by every other days by one time (3 times) is smaller than the number of metastatic nodules of KB/CV cell.

These results are obviously showing that 2-deoxy-L-ribose is perfectly suppressing the TP-dependent metastasis of tumor from spleen to liver.

### INDUSTRIAL APPLICABILITY

As mentioned above, by the present invention, the excellent effect that the TP-associated angiogenesis and metastasis of tumor can be perfectly suppressed by 2-deoxy-L-ribose or others is provided. And the agent whose effective component is 2-deoxy-L-ribose or others is useful as an inhibitor for angiogenesis, growth, invasion or metastasis of tumor.

## Claims

1. An inhibitor for angiogenesis comprising, as an active ingredient, at least one kind of compound selected from the group consisting of 2-deoxy-L-ribose, 2-deoxy-L-ribose derivatives whose hydroxyl group is methylated and/or acylated and 2-deoxy-D-ribose derivatives whose hydroxyl group is methylated and/or acylated.

2. A pharmaceutical formulation comprising, as an effective ingredient for inhibiting function of angiogenesis, at least one kind of compound selected from the group consisting of 2-deoxy-L-ribose, 2-deoxy-L-ribose derivatives whose hydroxyl group is methylated and/or acylated and 2-deoxy-D-ribose derivatives whose hydroxyl group is methylated and/or acylated.

3. A formulation for supressing growth, invasion or metastasis of tumor comprising, as an effective ingradient for inhibiting function of angiogenesis, at least one kind of compound selected from the group consisting of 2-deoxy-L-ribose, 2-deoxy-L-ribose derivatives whose hydroxyl group is methylated and/or acylated and 2-deoxy-D-ribose derivatives whose hydroxyl group is methylated and/or acylated.
